(19) Europäisches Patentamt
European Patent Office
Office européen des brevets

(11) Veröffentlichungsnummer: **0 467 177 A1**

# (12) EUROPÄISCHE PATENTANMELDUNG

(21) Anmeldenummer: **91111279.5**

(51) Int. Cl.⁵: **C07C 233/83**, G03F 7/039

(22) Anmeldetag: **06.07.91**

(30) Priorität: **18.07.90 DE 4022740**

(43) Veröffentlichungstag der Anmeldung: **22.01.92 Patentblatt 92/04**

(84) Benannte Vertragsstaaten: **BE DE FR GB IT**

(71) Anmelder: **BASF Aktiengesellschaft**
**Carl-Bosch-Strasse 38**
**W-6700 Ludwigshafen(DE)**

(72) Erfinder: **Funhoff, Dirk, Dr.**
**Panoramastrasse 3 a**
**W-6900 Heidelberg(DE)**
Erfinder: **Roser, Joachim, Dr.**
**Saarlandstrasse 40**
**W-6700 Ludwigshafen(DE)**
Erfinder: **Schulz, Guenther, Dr.**
**Im Roehrich 45**
**W-6702 Bad Duerkheim(DE)**

## (54) Mit Carbonylamino-N-alkancarbonsäure-Gruppen substituierte Vinylaromaten und ihre Verwendung.

(57) Die neuen, mit Carbonylamino-N-alkancarbonsäure-Gruppen substituierten Vinylaromaten der allgemeinen Formel I

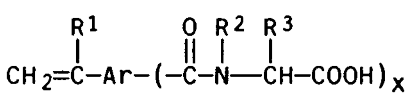

(I),

worin der Index und die Variablen die in den Ansprüchen und in der Beschreibung näher erläuterte Bedeutung haben, können in der Reprographie verwendet werden. Vorzugsweise dienen sie der Herstellung neuer, mit Carbonylamino-N-alkancarbonsäure-Gruppen substituierter Vinylaromat-(Co)-Polymerisate, welche für die Herstellung lichtempfindlicher Aufzeichnungsschichten (B) von lichtempfindlichen Aufzeichnungselementen verwendet werden können. Die neuen lichtempfindlichen Aufzeichnungselemente, deren neue lichtempfindliche Aufzeichnungsschichten (B) diese neuen Vinylaromat-(Co)Polymerisate enthalten oder hieraus bestehen, liefern durch bildmäßiges Belichten mit aktinischem Licht und Auswaschen (Entwickeln) mit wäßrig-alkalischen Entwicklerlösungsmitteln alkaliresistente Druckplatten und Photoresiste. Darüber hinaus eignen sich die neuen lichtempfindlichen Aufzeichnungselemente noch für die Herstellung positiver Abbildungen von Negativvorlagen.

EP 0 467 177 A1

Rank Xerox (UK) Business Services

Die vorliegende Erfindung betrifft neue, mit Carbonylamino-N-alkancarbonsäure-Gruppen substituierte Vinylaromaten der allgemeinen Formel I (Vinylaromaten I)

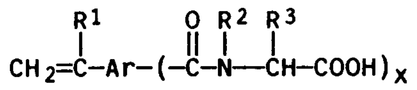

(I),

worin der Index und die Variablen die folgende Bedeutung haben:

$R^1$ Wasserstoffatom oder Methylgruppe;

Ar mindestens zweiwertiger, mit sonstigen organischen Resten substituierter oder unsubstituierter, einkerniger oder mehrkerniger aromatischer und heteroaromatischer Rest;

$R^2$ Wasserstoffatom, $C_1$-$C_6$-Alkyl, Cyclopentyl, Cyclohexyl und Phenyl;

$R^3$ Wasserstoffatom, sofern der Index x größer als 1 ist und/oder die Variable Ar keinen Phen-1,4-ylen-Rest bezeichnet;

$C_1$-$C_{10}$-Alkyl, substituiertes $C_1$-$C_{10}$-Alkyl und einwertiger, substituierter oder unsubstituierter, einkerniger oder mehrkerniger, aromatischer und heterocyclischer Rest;

$R^2$ und $R^3$ Bestandteil eines cyclisch verknüpften Rests; und

x ganze Zahl von 1 bis 3.

Im folgenden werden diese neuen, mit Carbonylamino-N-alkancarbonsäure-Gruppen substituierte Vinylaromaten der allgemeinen Formel I der Kürze halber als "Vinylaromaten I" bezeichnet.

Desweiteren betrifft die vorliegende Erfindung neue Vinylaromat-(Co)-Polymerisatela, welche die Vinylaromaten I einpolymerisiert enthalten.

Außerdem betrifft die vorliegende Erfindung die Verwendung dieser Vinylaromat-(Co)Polymerisate Ia sowie der Vinylaromat-(Co)Polymerisate Ib, welche N-(p-Vinylbenzoyl)-glycin einpolymerisiert enthalten, in der Reprographie zur Herstellung lichtempfindlicher Aufzeichnungselemente und alkaliresistenter Druckplatten und Photoresiste.

Die Herstellung und die Eigenschaften von N-(p-Vinylbenzoyl)-glycin und von N-(p-Vinylbenzoyl)-glycylglycin

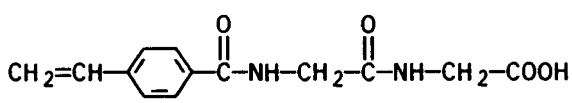

sowie ihre Umsetzung zu Vinylaromat-(Co)Polymerisaten Ib, welche N-(p-Vinylbenzoyl)-glycin oder -glycylglycin einpolymerisiert enthalten, sind aus den Publikationen von

- "Polymères porteurs de greffons peptidiques Ia, synthèse de styrène para-substitué par des greffons oligopeptide à extremités carboxyliques" von A. Nganié et al. in European Polymer Journal, Band 22, Nummer 6, Seiten 431 bis 438, 1986, und
- "Polymères porteurs de greffons peptidiques, polymérisation et copolymérisation de monomères styréniques para-substitués par des oligopeptides" von J.C. Brosse et al. in European Polymer Journal, Band 19, Seiten 55 bis 61, 1983,

bekannt. In diesen Publikationen wird die Verwendung von N-(p-Vinylbenzoyl)-glycin oder -glycylglycin sowie der hieraus hergestellten Vinylaromat-(Co)Polymerisate Ib in der Reprographie zur Herstellung lichtempfindlicher Aufzeichnungselemente und alkaliresistenter Druckplatten und Photoresiste nicht angesprochen.

Die Herstellung und die Eigenschaften einer Reihe von N-Benzoyl-α-aminosäuren sind aus der Publikation

- "Preparation of N-Benzoylamines by Photodecarboxylation of N-Benzoyl-α-amino Acids" von H. Nakai et al. in Synthesis, 1982, Seiten 141 bis 143,

bekannt. Diese bekannten N-Benzoyl-α-aminosäuren können bei Bestrahlen mit ultraviolettem Licht unter Bildung von N-Benzoylaminen decarboxylieren. Allerdings eignen sich diese bekannten N-Benzoyl-α-aminosäuren nicht für die Herstellung von Vinylaromat-(Co)Polymerisaten Ia oder Ib.

Aus der DE-A-38 25 738 ist ein lichtempfindliches Aufzeichnungselement für die Herstellung von Offsetdruckplatten bekannt, dessen lichtempfindliche Aufzeichnungsschicht ein Polymer mit einem mindestens eine Carboxylgruppe enthaltenden Rest enthält, welcher bei Belichtung mit aktinischem Licht decarboxyliert werden kann. Nach der bildmäßigen Photodecarboxylierung können die unbelichteten und

daher nicht decarboxylierten Bereiche dieser bekannten lichtempfindlichen Aufzeichnungsschicht mit Hilfe wäßrig-alkalischer Entwicklerlösungsmittel ausgewaschen (entwickelt) werden.

Der die Carboxylgruppe enthaltende Rest entspricht der allgemeinen Formel II.

$-(-Y-)_l-X-(-CH_2-)_k-COOH$ II

Hierin bedeuten

X -O-, -S- oder eine Einfachbindung oder eine Gruppe

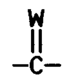

oder

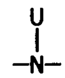 ,

wobei

W ein Sauerstoff- oder Schwefelatom und

U ein Wasserstoffatom oder eine substituierte oder unsubstituierte Aryl- oder Alkylgruppe ist;

Y ein substituiertes oder unsubstituiertes Alkylen, Arylen, Aralkylen oder eine divalente heterocyclische Gruppe;

k und l unabhängig voneinander 0 oder 1, und falls k = l = 0 ist, bedeutet X

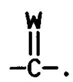

Demnach gehen aus der DE-A-38 25 738 keine Carbonylamino-N-alkancarbonsäure-Gruppen hervor, wie sie in den eingangs definierten Vinylaromaten I und den Vinylaromat-(Co)Polymerisaten Ia enthalten sein sollen.

Nachteilig für dieses bekannte lichtempfindliche Aufzeichnungselement bzw. für dessen lichtempfindliche Aufzeichnungsschicht (B) ist, daß es mit einem Belichtungsspielraum von 5 bis 10 Minuten belichtet werden muß, um einwandfreie Abbildungsergebnisse zu erzielen. Eine solche Belichtungszeit ist indes für die betriebliche Praxis einer Offset-Druckerei zu lang. Außerdem eignet sich dieses bekannte lichtempfindliche Aufzeichnungselement bzw. dessen lichtempfindliche Aufzeichnungsschicht (B) nicht sonderlich gut für die Herstellung positiver Abbildungen von Negativ- oder anderen Bildvorlagen.

Aufgabe der vorliegenden Erfindung war es, neue, einfach herstellbare substituierte Vinylaromaten aufzuzeigen, in welchen mindestens ein Rest mit mindestens einer photodecarboxylierbaren Carboxylgruppe vorliegt und welche sich für die Herstellung von Vinylaromat-(Co)Polymerisaten eignen, welche Reste mit jeweils mindestens einer photodecarboxylierbaren Carboxylgruppe enthalten. Darüber hinaus war es die Aufgabe der Erfindung, neue Vinylaromat-(Co)Polymerisate zu finden, in welchen seitenständige Reste mit mindestens einer photodecarboxylierbaren Carboxylgruppe enthalten sind und welche sich für die Herstellung neuer lichtempfindlicher Aufzeichnungselemente eignen. Eine weitere Aufgabe der vorliegenden Erfindung war es, neue lichtempfindliche Aufzeichnungselemente zu finden, in welchen die bei der bildmäßigen Belichtung mit aktinischem Licht stattfindende Differenzierung der lichtempfindlichen Aufzeichnungsschicht (B) in Bereiche, welche in wäßrig-alkalischen Entwicklerlösungsmitteln löslich, und in Bereiche, welche hierin nicht löslich sind, durch die Photodecarboxylierung hervorgerufen oder verstärkt wird.

Im Hinblick auf den Stand der Technik war es überraschend, daß all diese Aufgaben mit Hilfe der eingangs definierten, mit Carbonylamino-N-alkancarbonsäure-Gruppen substituierten Vinylaromaten der allgemeinen Formel I (Vinylaromaten I) gelöst werden konnten. Außerdem stand es im Hinblick auf den Stand der Technik nicht zu erwarten, daß die mit Hilfe der eingangs definierten Vinylaromaten I hergestellten neuen Vinylaromat-(Co)Polymerisate Ia derart vorteilhafte Eigenschaften aufweisen würden, daß sie in

hervorragender Weise für die Herstellung lichtempfindlicher Aufzeichnungselemente geeignet sind. Darüber hinaus stand es nicht zu erwarten, daß sich diese neuen lichtempfindlichen Aufzeichnungselemente wiederum außer zur Herstellung von negativen Abbildungen auch noch für die Herstellung von positiven Abbildungen von Negativ- oder sonstigen Bildvorlagen eignen würden.

Gegenstand der Erfindung sind dementsprechend die eingangs definierten neuen Vinylaromaten I.

In der allgemeinen Formel I steht die Variable $R^1$ für ein Wasserstoffatom oder eine Methylgruppe. Erfindungsgemäß ist es von Vorteil, wenn $R^1$ ein Wasserstoffatom bezeichnet.

In der allgemeinen Formel I steht die Variable Ar für einen mindestens zweiwertigen, mit sonstigen organischen Resten substituierten oder unsubstituierten, einkernigen oder mehrkernigen aromatischen oder heteroaromatischen Rest.

Hierbei bezeichnet "sonstiger organischer Rest" einen üblichen und bekannten organischen Rest, bei welchem es sich nicht um eine erfindungsgemäß zu verwendende Carbonylamino-N-alkancarbonsäure-Gruppe handelt, wie z.B. eine Methyl-, Nitro-, Cyan-, Ester-, Ether- oder Carboxylgruppe.

Erfindungsgemäß sind die mit sonstigen organischen Resten unsubstituierten Variablen Ar von Vorteil.

Beispiele vorteilhafter Variabler Ar dieser Art sind die Reste Ar-1 bis Ar-13

Ar-1 ,

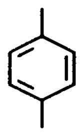

Ar-2 ,

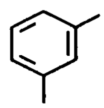

Ar-3 ,

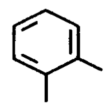

Ar-4 ,

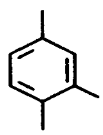

Ar-5 ,

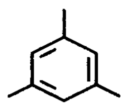

Ar-6 ,

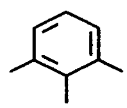

Ar-7 ,

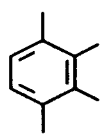

Ar-8 ,

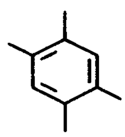

Ar-9 ,

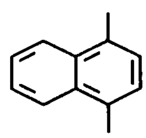

Ar-10 ,

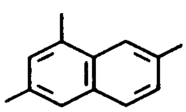

Ar-11 ,

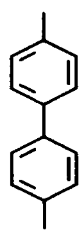

Ar-12

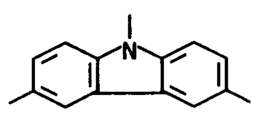

und

Ar-13 .

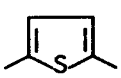

Von diesen sind die Reste Ar-1, Ar-2, Ar-10, Ar-11 und Ar-12 besonders und der Rest Ar-1 ganz besonders vorteilhaft.

In der allgemeinen Formel I steht $R^2$ für ein Wasserstoffatom, einen $C_1$-$C_6$-Alkylrest, einen Cyclopentylrest, einen Cyclohexylrest oder einen Phenylrest.

Beispiele erfindungsgemäß geeigneter $C_1$-$C_6$-Alkylreste $R^2$ sind die Methyl-, Ethyl-, n-Propyl-, i-Propyl-, n-Butyl-, i-Butyl-, n-Pentyl- und n-Hexylreste.

In der allgemeinen Formel I kann die Variable $R^3$ für ein Wasserstoffatom stehen, wenn der Index x der allgemeinen Formel I größer als 1 ist. Die Variable $R^3$ kann auch dann ein Wasserstoffatom bezeichnen, wenn die Variable Ar nicht für einen Phen-1,4-ylenrest (Ar-1) steht. Selbstverständlich kann die Variable $R^3$ auch für ein Wasserstoffatom stehen, wenn die beiden vorstehend genannten Bedingungen zugleich erfüllt sind.

Außerdem kann die Variable $R^3$ für einen unsubstituierten $C_1$-$C_{10}$-Alkylrest, einen substituierten $C_1$-$C_{10}$-Alkylrest oder einen einwertigen, substituierten oder unsubstituierten, einkernigen oder mehrkernigen, aromatischen oder heterocyclischen Rest stehen.

Beispiele erfindungsgemäß geeigneter unsubstituierter $C_1$-$C_{10}$-Reste $R^3$ sind Methyl-, Ethyl-, n-Propyl-, i-Propyl-, n-Butyl-, i-Butyl-, sec.-Butyl-, tert.-Butyl-, n-Pentyl-, Neopentyl-, n-Hexyl-, n-Heptyl-, n-Octyl-, 2-Ethylhexyl-, n-Nonyl-, 3,5,5-Trimethylhexyl- und n-Decylreste. Von diesen sind diejenigen Reste $R^3$ vorteilhaft, welche in den aliphatischen Aminosäuren Alanin, Valin, Leucin und Isoleucin vorkommen.

Beispiele geeigneter substituierter $C_1$-$C_{10}$-Alkylreste sind die vorstehend genannten Alkylreste, welche stickstoff-, sauerstoff-, schwefel- und/oder phosphorhaltige funktionelle Gruppen und/oder gesättigte oder ungesättigte aliphatische, aromatische und/oder heterocyclische Reste tragen.

Beispiele erfindungsgemäß vorteilhafter substituierter $C_1$-$C_{10}$-Alkylreste sind die Reste, wie sie in den Aminosäuren Serin, Phenylserin, Threonin, Cystein, Cystin, Methionin, Lanthionin, Ornithin, Citrullin, Arginin, Asparaginsäure, Asparagin, Glutaminsäure, Glutamin, Phenylalanin, Tyrosin, 3,5-Diiod-tyrosin, Tyroxin, 3,4-Dihydroxy-phenylalanin, Tryptophan und Histidin vorkommen.

Außerdem kann die Variable $R^3$ in der allgemeinen Formel I für einen einwertigen, substituierten oder unsubstituierten, einkernigen oder mehrkernigen, aromatischen oder heterocyclischen Rest stehen.

Beispiele erfindungsgemäß geeigneter Variablen $R^3$ sind die nachfolgend beschriebenen Reste $R^3$-1 bis $R^3$-11.

$R^3$-1, 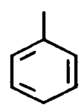

$R^3$-2, 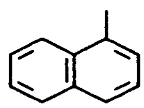

$R^3$-3, 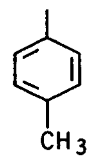

$R^3$-4, 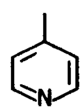

$R^3$-5, 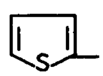

$R^3$-6, 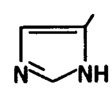

$R^3$-7, 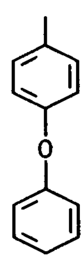

$R^3$-8, 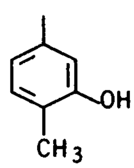

$R^3$-9, 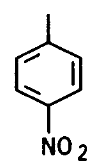

$R^3$-10 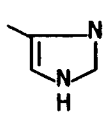 und

$R^3$-11. 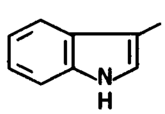

Die Variablen $R^2$ und $R^3$ können indes auch Bestandteil eines cyclischen verknüpften Rests sein, wie beispielsweise der Reste ($R^2R^3$)-1 bis ($R^2R^3$)-5:

$(R^2R^3)$-1,

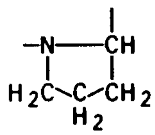

$(R^2R^3)$-2,

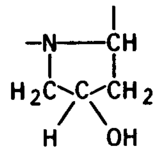

$(R^2R^3)$-3,

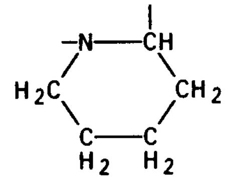

$(R^2R^3)$-4

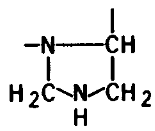

und

$(R^2R^3)$-5.

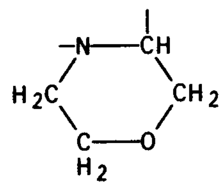

Von diesen sind die Reste $(R^2R^3)$-1 und $(R^2R^3)$-2 besonders vorteilhaft.

In der allgemeinen Formel I steht der Index x für eine ganze Zahl von 1 bis 3. Erfindungsgemäß ist es von Vorteil, wenn der Index x gleich 1 ist.

Beispiele vorteilhafter neuer Vinylaromaten I sind die neuen Vinylaromaten I-1 bis I-26:

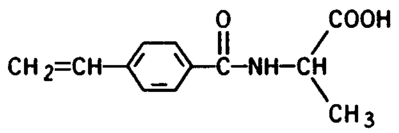

I-1,

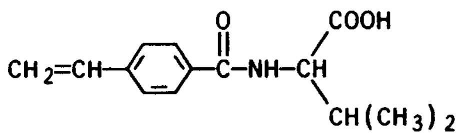

I-2,

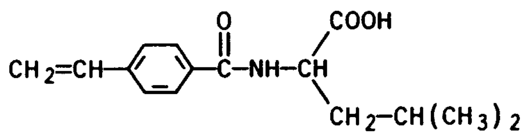

I-3,

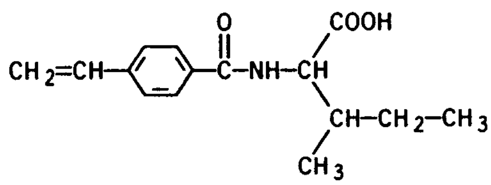

I-4,

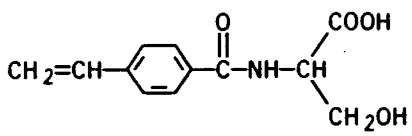

I-5,

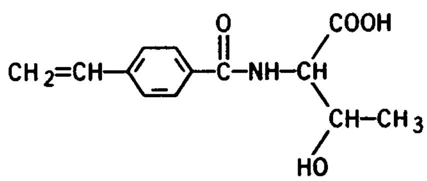

I-6,

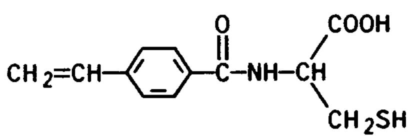

I-7,

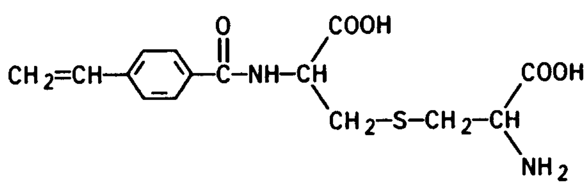

I-8,

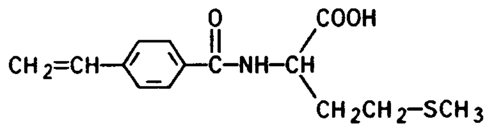
I-9,

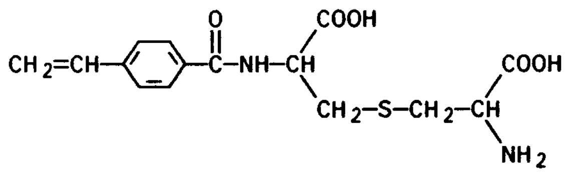
I-10,

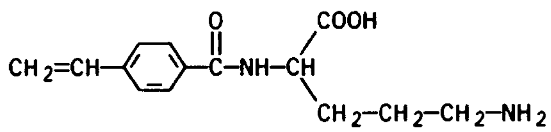
I-11,

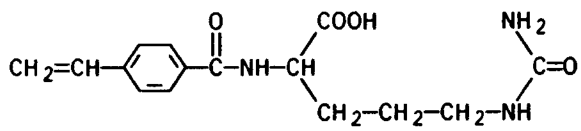
I-12,

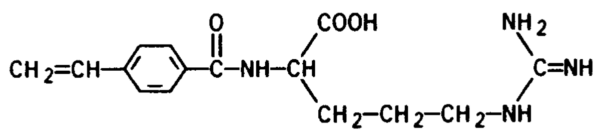
I-13,

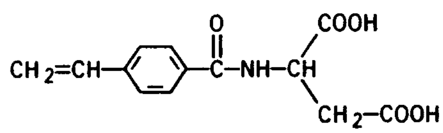
I-14,

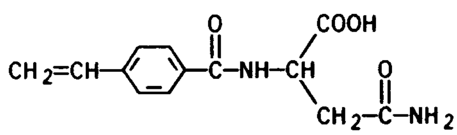
I-15,

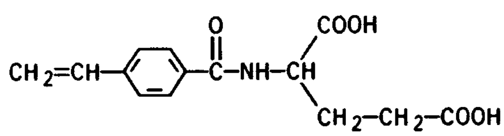
I-16,

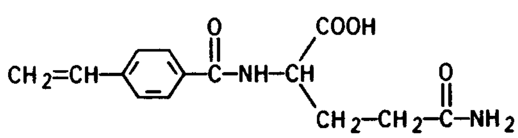
I-17,

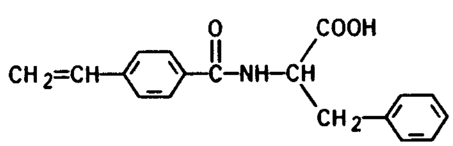
I-18,

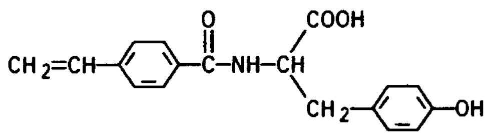

I-19,

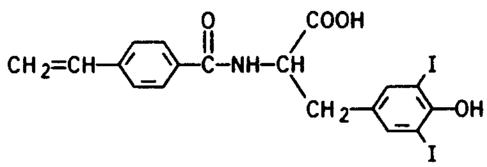

I-20,

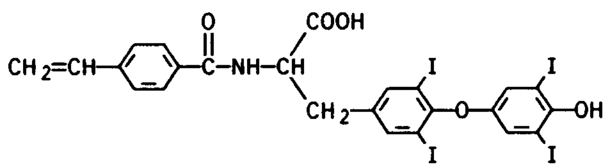

I-21,

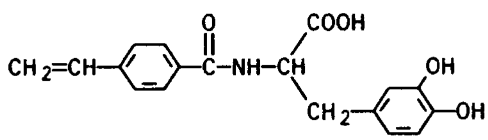

I-22,

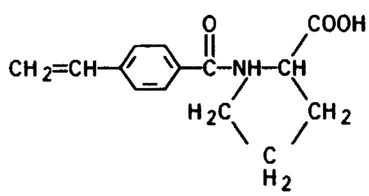

I-23,

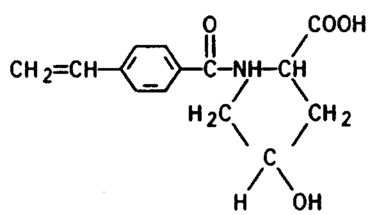

I-24,

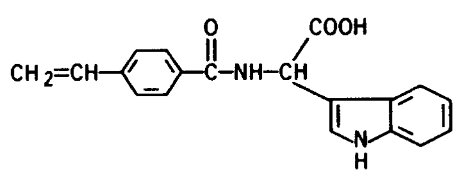

I-25 und

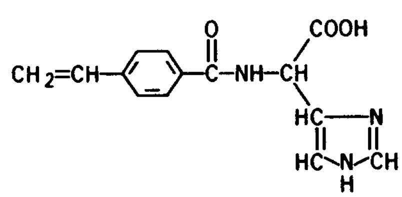

I-26.

Von diesen werden die Vinylaromaten I-1, I-3 und I-18 bevorzugt verwendet, weil sie erfindungsgemäß besonders vorteilhaft sind.

Die Herstellung der neuen Vinylaromaten I kann nach den üblichen und bekannten Methoden der präparativen organischen Chemie erfolgen. Vorteilhafterweise geht man hierbei aus von einem mit ein bis drei Halogenformylgruppen substituierten Vinylaromaten oder -heteroaromaten, welchen man in üblicher

und bekannter Weise mit einer Aminocarbonsäure umsetzt. Hierbei können sonstige funktionelle Gruppen in der betreffenden Aminocarbonsäure, welche unter Umständen die Bildung der Amidgruppe stören würden, in geeigneter Weise, z.B. durch Veresterung, Veretherung oder Amidierung, blockiert werden. Für die Herstellung der neuen Vinylaromaten I nach dem genannten Verfahren eignen sich insbesondere die Aminocarbonsäuren Alanin, Valin, Leucin, Isoleucin, Serin, Threonin, Cystein, Cystin, Methionin, Lanthionin, Ornithin, Citrullin, Arginin, Asparaginsäure, Asparagin, Glutaminsäure, Glutamin, Phenylalanin, Tyrosin, 3,5-Diiod-tyrosin, Tyroxin, 3,4-Dihydroxyphenylalanin, Prolin, Hydroxyprolin, Tryptophan und Histidin sowie deren Derivate, in welchen die sonstigen funktionellen Gruppen in geeigneter Weise blockiert sind.

Desweiteren können die neuen Vinylaromaten I durch Umsetzen eines mit ein bis drei Carboxylgruppen substituierten Vinylaromaten mit Halogenwasserstoffaddukten veresterter Aminocarbonsäuren hergestellt werden. Hierfür kommen insbesondere die Ethylester der vorstehend genannten Aminocarbonsäuren in Betracht. Nach der Bildung der Amidgruppen können diese Estergruppen wieder in üblicher und bekannter Weise gespalten werden, wodurch die neuen Vinylaromaten I resultieren.

Die neuen Vinylaromaten I können als solche in der Reprographie als photopolymerisierbare und/oder photodecarboxylierbare Monomere verwendet werden, wobei sie in dieser Funktion Vorteile gegenüber dem Stand der Technik aufweisen, weil sie die Möglichkeit eröffnen, über die Abspaltung ihrer Carbonsäuregruppen bei der Bestrahlung mit aktinischem Licht (Photodecarboxylierung), die Löslichkeitsdifferenzierung zwischen den bildmäßig belichteten und nicht belichteten Bereichen einer lichtempfindlichen Aufzeichnungsschicht noch zu verstärken.

Insbesondere eignen sich die neuen Vinylaromaten I für die Herstellung der neuen Vinylaromat-(Co)-Polymerisate Ia.

Die neuen Vinylaromat-(Co)Polymerisate Ia enthalten mindestens eines der neuen Vinylaromaten I einpolymerisiert. Demgemäß enthalten die neuen Vinylaromat-(Co)Polymerisate Ia mindestens eine seitenständige Carbonylamino-N-alkancarbonsäure-Gruppe, welche über die Variable Ar mit der Polymerhauptkette verbunden ist. Erfindungsgemäß ist es indes von Vorteil, wenn die neuen Vinylaromat-(Co)Polymerisate Ia mehr als eine Carbonylamino-N-alkancarbonsäure-Gruppe enthalten.

Demgemäß kann es sich bei den neuen Vinylaromat-(Co)Polymerisaten Ia um neue Vinylaromat-Homopolymerisate Ia handeln, welche nur eine Art der neuen Vinylaroamten I einpolymerisiert enthalten. Oder aber es handelt sich um neue Vinylaromat-Copolymerisate Ia, welche mehr als eine Art der neuen Vinylaromaten I einpolymerisiert enthalten. Daneben kann es sich bei den neuen Vinylaromat-Copolymerisaten Ia noch um solche handeln, welche mindestens eine Art der neuen Vinylaromaten I und mindestens ein an sich bekanntes Monomeres einpolymerisiert enthalten. Bei diesen üblichen und bekannten Monomeren kann es sich um Vinylaromaten wie Styrol, p-Methylstyrol, α-Methylstyrol, Vinylnaphthalin und/oder N-Vinylcarbazol; (Meth)-Acrylate wie Methyl-, Ethyl-, n-Propyl-, i-Propyl-, n-Butyl-, i-Butyl-, sec.-Butyl-, tert.-Butyl-, n-Pentyl-, i-Pentyl-, Neopentyl-, Cyclopentyl-, n-Hexyl-, Cyclohexyl-, 4-Methyl-cyclohexyl-, n-Heptyl-, n-Octyl-, 2-Ethylhexyl-, i-Octyl-, n-Nonyl-, 3,3,5-Trimethylhexyl-, 2-Hydroxyethyl-, 3-Hydroxy-propyl-, 2-Hydroxy-propyl-, Glycerinmono-, Triethylenglykol-mono-, Tetraethylenglykol-mono- und/oder Pentaerythritol-mono(meth)acrylat; (Meth)Acrylamide wie (Meth)Acrylamid und/oder N-Methylol-, N-Methyl-, N,N-Dimethyl-, N-Ethyl-, N,N-Diethyl-, N-n-Butyl-, N,N-Di-n-butyl-, N-n-Hexyl-, N,N-Di-n-hexyl-, N-Phenyl- und/oder N-(4-Hydroxyphenyl(meth)acrylamid; Vinylester wie Vinylacetat und/oder Vinylpropionat; (Meth)Acrylsäure; (Meth)Acrylnitril; Allylether; Allylester; und/oder um Vinylether wie n-Butylvinylether handeln.

Die Herstellung der neuen Vinylaromat-(Co)Polymerisate Ia weist keine methodischen Besonderheiten auf sondern erfolgt in üblicher und bekannter Weise durch radikalische Homo- oder Copolymerisation eines der neuen Vinylaromaten I für sich alleine, zweier oder mehrerer neuer Vinylaromaten I miteinander oder mindestens eines neuen Vinylaromaten I mit mindestens einem an sich bekannten Monomeren, wie beispielsweise die vorstehend genannten Monomeren oder zusätzlich auch noch N-(p-Vinylbenzoyl)-glycin.

Erfindungsgemäß von besonderem Vorteil sind diejenigen neuen Vinylaromat-(Co)Polymerisate Ia, welche die vorstehend beschriebenen neuen Vinylaromaten I-1 bis I-26 einpolymerisiert enthalten. Hierbei erweisen sich diejenigen neuen Vinylaromat-(Co)Polymerisate als ganz besonders vorteilhaft, welche die neuen Vinylaromaten 1-1, 1-3 und/oder 1-18 einpolymerisiert enthalten.

Die neuen Vinylaromat-(Co)Polymerisate Ia können aufgrund ihrer besonderen, vorteilhaften Eigenschaften zahlreichen Anwendungszwecken zugeführt werden. So können sie als Membranmaterialien für die Entsalzung von Wasser oder als Trägermaterialien für aufgepfropfte Proteine und Enzyme dienen. Vor allem aber können sie in der Reprographie angewandt werden. Hierbei werden sie vorteilhafterweise in den lichtempfindlichen Aufzeichnungsschichten (B) von lichtempfindlichen Aufzeichnungselementen, welche der Herstellung von Druckplatten und Photoresisten dienen, verwendet. Für diesen Anwendungszweck können sie gemeinsam mit den an sich bekannten Vinylaromat-(Co)Polymerisaten Ib, welche N-(p-Vinylbenzoyl)-glycin einpolymerisiert enthalten, verwendet werden. Indes kommen für diesen Anwendungszweck in

speziellen Fällen die Vinylaromat-(Co)Polymerisate Ib alleine in Betracht.

Erfindungsgemäß von Vorteil ist es, wenn die neuen Vinylaromat-(Co)Polymerisatela und/oder die Vinylaromat-(Co)Polymerisate Ib in lichtempfindlichen Aufzeichnungselementen für die Herstellung von Druckplatten und Photoresisten verwendet werden, welche

A) einen dimensionsstabilen Träger und

B) eine lichtempfindliche Aufzeichnungsschicht, deren unbelichteten Bereiche nach der bildmäßigen Belichtung mit aktinischem Licht mit wäßrig-alkalischen Entwicklerlösungsmitteln ausgewaschen (entwickelt) werden können,

enthalten.

Hierbei ist es erfindungsgemäß ganz besonders vorteilhaft, wenn es sich bei den lichtempfindlichen Aufzeichnungselementen um lichtempfindliche negativ arbeitende Offset-Druckplatten handelt.

Diese lichtempfindlichen negativ arbeitenden Offset-Druckplatten können bekanntermaßen

(A) einen offsetplattentypischen dimensionsstabilen Träger aus Aluminium oder einer Aluminiumlegierung und

(B) eine lichtempfindliche, negativ arbeitende mit wäßrig-alkalischen Entwicklerlösungsmitteln entwickelbare Aufzeichnungsschicht, welche

$b_1$) mindestens ein in den wäßrig-alkalischen Entwicklerlösungsmitteln lösliches oder dispergierbares polymeres Bindemittel, $b_2$) mindestens ein Diazoniumsalz-Gruppen enthaltendes Harz, hergestellt aus einem Aryl-Diazoniumsalz; und

$b_3$) Hilfsstoffe

enthält,

enthalten.

Lichtempfindliche negativ arbeitende Offset-Druckplatten dieser Art sind beispielsweise aus der EP-A-0 345 636 und der EP-A-0 177 962 bekannt.

Bei dieser Anwendung erweist sich ein überraschender Vorteil der neuen Vinylaromat-(Co)Polymerisate Ia und der bekannten Vinylaromat-(Co)Polymerisate Ib: sie können in diesen bekannten lichtempfindlichen negativ arbeitenden Offset-Druckplatten das jeweils verwendete polymere Bindemittel ($b_1$) zum Teil oder völlig ersetzen. Darüber hinaus ist es noch möglich, nicht nur die polymeren Bindemittel ($b_1$) durch die neuen Vinylaromat-(Co)Polymerisate Ia und/oder die bekannten Vinylaromat-(Co)Polymerisate Ib zu ersetzen, sondern auch die Diazonium-Gruppen enthaltenden Harze ($b_2$). Hieraus resultiert dann ein lichtempfindliches Aufzeichnungselement bzw. eine lichtempfindliche negativ arbeitende Offset-Druckplatte, welche der in der DE-A-38 25 738 beschriebenen überlegen ist.

Die neuen lichtempfindlichen Aufzeichnungselemente, welche man unter Verwendung der neuen Vinylaromat-(Co)Polymerisate Ia und/oder der an sich bekannten Vinylaromat-(Co)Polymerisate Ib hergestellt hat, können, wie beispielsweise in der EP-A-0 345 636, der EP-A-0 177 962 oder der DE-A-38 25 738 beschrieben, durch bildmäßiges Belichten mit aktinischem Licht und Entwickeln mit einem üblichen und bekannten wäßrig-alkalischen Entwicklerlösungsmittel zu Druckplatten und Photoresisten, insbesondere aber zu Offset-Druckplatten, verarbeitet werden. Hierbei erweisen sich die neuen lichtempfindlichen Aufzeichnungselemente, insbesondere aber die neuen lichtempfindlichen negativ arbeitenden Offset-Druckplatten, als ganz besonders vorteilhaft, denn sie können nach ihrer Herstellung in einfacher Weise durch vollflächiges Nachbelichten mit aktinischem Licht in ihrer Abriebfestigkeit, Beständigkeit gegenüber wäßrigen Medien, insbesondere gegenüber dem Feuchtwasser von Offset-Druckfarben, ihrer Überzugsfestigkeit und ihrer Haftung an dem dimensionsstabilen Träger (A) entscheidend verbessert werden. Bei den aus den neuen lichtempfindlichen Aufzeichnungselementen hergestellten Offset-Druckplatten resultiert durch diese Nachbelichtung außerdem noch eine verbesserte Farbführung der druckenden Bereiche.

Die neuen lichtempfindlichen Aufzeichnungselemente können indes nicht nur der Erzeugung negativer Abbildungen von Negativ- oder sonstigen Bildvorlagen, sondern auch der Herstellung positiver Abbildungen dienen. Dies ist ein nicht zu unterschätzender Vorteil, können doch die neuen lichtempfindlichen Aufzeichnungselemente deshalb auch als sogenannte "Umkehrplatten" verwendet werden, welche sich zugleich für die negative und die positive Wiedergabe von Bildvorlagen eignen und welche deshalb für die reprographische Praxis von erheblicher Bedeutung sind.

Für die Herstellung positiver Abbildungen von Negativ- oder sonstigen Bildvorlagen wird das neue lichtempfindliche Aufzeichnungselement zunächst in üblicher und bekannter Weise mit aktinischem Licht bildmäßig belichtet und hiernach mittels eines organischen Entwicklerlösungsmittels entwickelt, wobei nur die belichteten Bereiche der bildmäßig belichteten Aufzeichnungsschicht (B) des neuen lichtempfindlichen Aufzeichnungselements weggewaschen werden. Dadurch resultiert die gewünschte positive Abbildung der Vorlage. Hiernach wird das entwickelte Aufzeichnungselement vollflächig mit aktinischem Licht nachbelichtet, wodurch sich auch hier die vorstehend bei der Beschreibung des negativen Abbildungsverfahrens näher erläuterten Verbesserungen und Vorteile einstellen.

Beispiel 1

Die Herstellung von p-Vinylbenzoylalanin I-1

Versuchsvorschrift:

Die Herstellung von p-Vinylbenzoylalanin I-1 erfolgte analog der in European Polymer Journal, Band 22, Nummer 6, Seiten 431 bis 438, 1986, von A. Nganie et al. angegebenen Vorschrift.

Hierzu wurden zunächst 10 g p-Vinylbenzoesäure (0,068 mol) mit 11,5 ml Thionylchlorid unter Inertgas während 2 Tagen bei Raumtemperatur zu p-Vinylbenzoesäurechlorid (bräunlich gefärbte Flüssigkeit) umgesetzt. Nach Beendigung der Reaktion wurde das überschüssige Thionylchlorid im Vakuum abdestilliert, und das resultierende p-Vinylbenzoesäurechlorid-Rohprodukt wurde direkt für die Synthese des erfindungsgemäßen Vinylaromaten I-1 verwendet.

Zu diesem Zweck wurden 6,1 g (0,068 mol) DL-Alanin in 75 ml 2N Natronlauge gelöst. Das resultierende Gemisch wurde 1 h bei 0 °C gehalten und anschließend langsam unter Rühren und unter Inertgas mit dem p-Vinylbenzoesäurechlorid-Rohprodukt versetzt. Nach einer Stunde Reaktionszeit bei 0 °C wurde das so erhaltene Reaktionsgemisch angesäuert, und der hierbei resultierende Niederschlag wurde abfiltriert, mit Wasser gewaschen und getrocknet.

Nach dem Umkristallisieren des getrockneten Produktes aus Ethylbenzol resultierte p-Vinylbenzoylalanin I-1 in der Form weißer Kristalle, deren Infrarot- und Kernresonanz-Spektren den theoretischen Erwartungen entsprachen und damit den Strukturvorschlag bestätigten. Desgleichen stimmte die elementaranalytisch ermittelte Zusammensetzung der Kristalle innerhalb der Fehlergrenzen mit den theoretisch berechneten Werten überein.

Das in dieser Weise erhaltene p-Vinylbenzoylalanin I-1 war hervorragend für die Herstellung der neuen Vinylaromat-Copolymerisate Ia geeignet.

Beispiel 2

Die Herstellung eines neuen Vinylaromat-Copolymerisats Ib unter Verwendung von p-Vinylbenzoylalanin I-1

Versuchsvorschrift:

Eine Mischung aus, bezogen auf die Mischung, 17 Gew.% p-Vinylbenzoylalanin I-1, 20 Gew.% Acrylnitril, 60 Gew.% Ethylacrylat und 3 Gew.% Methacrylsäure wurden in an sich bekannter Weise in Lösung radikalisch copolymerisiert (vgl. z.B. die Herstellung des "Polymer-1" der EP-A-0 177 962). Das hierbei erhaltene neue Vinylaromat-Copolymerisat Ia wies einen K-Wert nach Fikentscher (gemessen an einer 1 gew.%igen Lösung in Dimethylformamid) von 53,1 auf. Es war ganz vorzüglich für die Herstellung neuer lichtempfindlicher Aufzeichnungselemente geeignet.

Beispiel 3

Die Herstellung einer Offset-Druckplatte unter Verwendung des neuen Vinylaromat-Copolymerisats Ia

Versuchsvorschrift:

Auf ein in üblicher und bekannter Weise aufgerauhtes, anodisch oxidiertes und mit Silikat-Lösung behandeltes Aluminium-Trägerblech (A) für Offset-Druckplatten wurde eine Lösung, enthaltend die nachfolgend beschriebene Zusammensetzung, so aufgetragen, daß nach dem Trocknen eine lichtempfindliche Aufzeichnungsschicht (B) mit einem Schichtgewicht von 1.5 g/$m^2$ resultierte.

| | |
|---|---|
| 88.7 Gew.% | des neuen Vinylaromat-Copolymerisats Ia gemäß Beispiel 2 |
| 10.0 Gew.% | des Kondensationsproduktes von p-Diphenylamindiazoniumhexafluorphosphat mit Paraformaldehyd als Diazoharz |
| 1.0 Gew.% | Viktoria Reinblau (C.I. BB7) und |
| 0.3 Gew.% | Michlers Keton. |

Die resultierende lichtempfindliche Aufzeichnungsschicht (B) wurde anschließend 48 s lang durch ein

Standard-Negativ (UGRA-Testkeil) mit Hilfe eines handelsüblichen Belichters (Kopiergerät 650 BI der Firma Sack) belichtet.

Das belichtete Aufzeichnungselement wurde dann von Hand mit einer handels-üblichen wäßrig-alkalischen Entwicklerlösung vom pH-Wert 11 bis 11.5 (nylolith EN 10 der Firma BASF) unter intensivem Druck und starkem Reiben entwickelt.

Die erhaltene Offset-Druckplatte wies keinerlei Beschädigungen der Bildbereiche auf. Außerdem waren diese überhaupt nicht kratzempfindlich.

Vergleichsversuch V1

Die Herstellung einer Offsetdruckplatte unter Verwendung eines bekannten Copolymerisats

Versuchsvorschrift:

Beispiel 3 wurde wiederholt, nur daß anstelle der dort beschriebenen lichtempfindlichen Aufzeichnungsschicht (B) eine lichtempfindliche Aufzeichnungsschicht (B) folgender Zusammensetzung verwendet wurde:

| | |
|---|---|
| 88.7 Gew.% | eines Copolymerisats aus N-(4-Hydroxyphenyl)methacrylamid, Acrylnitril, Ethylacrylat und Methacrylsäure (Polymer-1 der EP-A-177 962) als Bindemittel, |
| 10.0 Gew.% | des Kondensationsproduktes von p-Diphenylamindiazoniumhexafluorophosphat mit Paraformaldehyd als Diazoharz, |
| 1.0 Gew.% | Viktoria Reinblau (C.I. BB7) und |
| 0.3 Gew.% | Michlers Keton. |

Nach der Entwicklung war bei der Offset-Druckplatte ein deutlicher Verlust von feinen Bildelementen festzustellen. Zudem waren ihre Bildbereiche kratzempfindlich.

**Patentansprüche**

**1.** Mit Carbonylamino-N-alkancarbonsäure-Gruppen substituierte Vinylaromaten der allgemeinen Formel I (Vinylaromaten I)

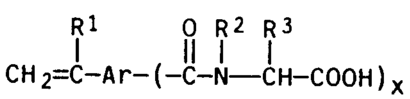

(I),

worin der Index und die Variablen die folgende Bedeutung haben:

| | |
|---|---|
| $R^1$ | Wasserstoffatom oder Methylgruppe; |
| Ar | mindestens zweiwertiger, mit sonstigen organischen Resten substituierter oder unsubstituierter, einkerniger oder mehrkerniger aromatischer und heteroaromatischer Rest; |
| $R^2$ | Wasserstoffatom, $C_1$-$C_6$-Alkyl, Cyclopentyl, Cyclohexyl und Phenyl; |
| $R^3$ | Wasserstoffatom, sofern der Index x größer als 1 ist und/oder die Variable Ar keinen Phen-1,4-ylen-Rest bezeichnet; $C_1$-$C_{10}$-Alkyl, substituiertes $C_1$-$C_{10}$-Alkyl und einwertiger, substituierter oder unsubstituierter, einkerniger oder mehrkerniger, aromatischer und heterocyclischer Rest; |
| $R^2$ | und $R^3$ Bestandteil eines cyclisch verknüpften Rests; und |
| x | ganze Zahl von 1 bis 3. |

**2.** Die Vinylaromaten I nach Anspruch 1, dadurch gekennzeichnet, daß die Variable Ar einen mit sonstigen organischen Resten substituierten oder unsubstituierten Phenylenrest bezeichnet, $R^1$ für ein Wasserstoffatom steht und x gleich 1 ist.

**3.** Vinylaromat-(Co)Polymerisate Ia, dadurch gekennzeichnet, daß sie Vinylaromaten I gemäß Anspruch 1 oder 2 einpolymerisiert enthalten.

**4.** Verwendung der Vinylaromat-(Co)Polymerisate Ia gemäß Anspruch 3 und von Vinylaromat-(Co)-Polymerisaten Ib, welche N-(p-Vinylbenzoyl)-glycin einpolymerisiert enthalten, in der Reprographie.

**5.** Lichtempfindliches Aufzeichnungselement für die Herstellung von Druckplatten und Photoresisten,

enthaltend

A) einen dimensionsstabilen Träger und

B) eine lichtempfindliche Aufzeichnungsschicht, deren unbelichteten Bereiche nach der bildmäßigen Belichtung mit aktinischem Licht mit wäßrig-alkalischen Entwicklerlösungsmitteln ausgewaschen (entwickelt) werden können,

dadurch gekennzeichnet, daß die lichtempfindliche Aufzeichnungsschicht (B) mindestens ein mit Carbonylamino-N-alkancarbonsäure-Gruppen substituiertes Vinylaromat-(Co)Polymerisat Ia gemäß Anspruch 3 und/oder mindestens ein Vinylaromat-(Co)Polymerisat Ib, welches N-(p-Vinylbenzoyl)-glycin einpolymerisiert enthält, aufweist oder aus mindestens einem der Vinylaromat-(Co)Polymerisate Ia und/oder Ib besteht.

Europäisches Patentamt

# EUROPÄISCHER RECHERCHENBERICHT

Nummer der Anmeldung

**EP 91 11 1279**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | CHEMICAL ABSTRACTS, Band 100, 1984, Seite 158, Zusammenfassung Nr. 46484x, Columbus, Ohio, US; A. WALDE et al.: "p-Cymene metabolism in rats and guinea pigs", & XENOBIOTICA 1983, 13(8), 503-12 * Zusammenfassung * & GENERIC DARC ONLINE BILD VON CHEMICAL ABSTRACT VERBINDUNGNUMMER 88416-63-1 | 1,3 | C 07 C 233/83<br>G 03 F 7/039 |
| D,A | DE-A-3 825 738 (FUJI) * Insgesamt * | 1 | |
| D,A | EUROPEAN POLYMER JOURNAL, Band 19, 1983, Seiten 55-61; J.C. BROSSE et al.: "Polymeres porteurs de greffons peptidiques * Insgesamt * | 1,3 | |
| D,A | EUROPEAN POLYMER JOURNAL, Band 22, 1986, Seiten 431-438; A. NGANIE et al.: "Polymères porteurs de greffons peptidiques-Ia" * Insgesamt * | 1,3 | RECHERCHIERTE SACHGEBIETE (Int. Cl.5) |
| D,A | EP-A-0 177 962 (MITSUBISHI) * Insgesamt * | 1,3 | C 07 C 233/00<br>C 07 D 333/00<br>G 03 F 7/00 |

Der vorliegende Recherchenbericht wurde für alle Patentansprüche erstellt

| Recherchenort | Abschlußdatum der Recherche | Prüfer |
|---|---|---|
| Den Haag | 18 Oktober 91 | WELLS A.G. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument